# EUROPEAN PATENT APPLICATION

(11) **EP 4 273 307 A1**
(43) Date of publication of application: **08.11.2023**
(21) Application number: 20967409.2
(22) Date of filing: 29.12.2020
(51) Int. Cl.: C40B 40/06, C40B 50/06, C40B 80/00

(54) **METHOD FOR RAPIDLY CONSTRUCTING CYCLIZED LIBRARY AND RING-FORMING LINKER**

(71) Applicant: MGI Tech Co., Ltd., Shenzhen, Guangdong 518083 (CN)
(72) Inventor: YU, Youqian, Shenzhen, Guangdong 518083 (CN); FU, Shujin, Shenzhen, Guangdong 518083 (CN); GENG, Chunyu, Shenzhen, Guangdong 518083 (CN); LIANG, Xinming, Shenzhen, Guangdong 518083 (CN)
(74) Representative: Habermann, Hruschka & Schnabel
(86) International application number: PCT/CN2020/140877
(87) International publication number: WO 2022/141061

(57) **Abstract**

The present invention falls within the field of biotechnology, and specifically discloses a method for constructing a cyclized library and a ring-forming linker. The method comprises: 1) breaking a DNA sequence into fragments; 2) enabling two ends of the broken fragment to form 3' end protrusions; and 3) cyclizing the fragment with 3' end protrusions to form a ring-shaped library by means of a ring-forming linker, wherein the ring-forming linker is a double chain which is not completely paired and has 3' end protrusion at both ends, and the 3' end protrusion of the ring-forming linker is complementary to the 3' end protrusion of the broken fragment. According to the present invention, an A-sticky end of the end is formed by means of repairing the end of and the adding A to the broken DNA fragment, and same is then complementary to the specifically designed linker T sticky end to form a cyclized structure, and the connection at the gap is completed under the action of a ligase.

## Description

### TECHNICAL FIELD

The present disclosure belongs to the field of biotechnology. More specifically, the present disclosure provides a method for rapidly constructing cyclized library and cyclization adapters for the method.

### BACKGROUND OF THE INVENTION

At present, there are many well-established library construction methods based on next-generation sequencing (NGS, also known as High-Throughput Sequencing) technology. However, these library construction methods are generally based on the conventional library construction process, which comprises the following steps: the genomic nucleic acid strands are fragmented by physical or enzymatic means; the obtained fragments are subjected to end-repair by exonuclease and two ends of the fragments are filled in to ensure both ends are blunt, and then A is added to the 3' end of the fragments by polymerase to generate a single-base sticky end; same adapters are added to both ends of the fragments with A at the 3' end. The adapter is formed by complementary pairing of a long nucleic acid strand and a short strand, and gap is ligated by ligase after complementary pairing of A and T; the fragment ligated with adapter is used as a template and a nucleic acid single-stranded primer complementary with the adapter is added as primer for Polymerase Chain Reaction (PCR); two single strands of the template are separated by denaturation, and after primer binding to the corresponding single strand, the corresponding single strand is extended to be a completely complementary double-stranded target product, with one end being A adapter and the other end being B adapter; the ligation products are purified and recycled by magnetic beads; the purified and recycled DNA double-stranded products are denatured to obtain single-stranded DNA; the target DNA single strands are cyclized by using a nucleic acid single-stranded primer for assisting cyclization and the screening of the phosphoric acid group at the 5' end of the DNA single strands; the unnecessary and remaining uncyclized single strands are removed by using exonuclease, and the like; the cyclized products are purified and recycled by magnetic beads; the cyclized single-stranded circular nucleic acid products are subjected to subsequent sequencing; DNA Nanoballs (DNBs) formed by rolling circle amplication are used for reading nucleic acid sequence information. This process involves several tube-changing and purification steps, which could be improved in terms of ease of operation and time of library construction.

Adapters used in NGS high-throughput sequencing should be a specially designed DNA sequence. The characteristic sequence in an adapter will function as the sequence of start site and be paired with sequencing primer during sequencing. The subsequent sequence can be determined through the extension of the primer. An adapter may be ligated to both ends of a DNA fragment by ligation and the like. The ligation of adapters with sticky ends is usually used to achieve directional ligation and avoid ligation between adapters. In the conventional library construction, it is necessary to ensure that adapters are ligated to both ends of DNA double strands and the redundant adapter products are removed by purification.

With the proceeding of national sequencing services for large-scale residents in many countries, there is a need for an easy-operating and time-saving method for constructing a cyclized library in the prior art.

### SUMMARY OF THE INVENTION

To solve the problems in the prior art, the present disclosure provides a method for rapidly constructing cyclized library and cyclization adapters for the method.

Therefore, on one aspect, the present disclosure provides a method for constructing cyclized library, the method comprising:
1) fragmenting a DNA sequence into fragments;
2) enabling two ends of the fragments to form 3' overhangs; and
3) cyclizing the fragments with 3' overhang to form a cyclized library by using a cyclization adapter, wherein the cyclization adapter is formed by incompletely complementary double strands and has a 5' overhang at both ends, and the 5' overhang of the cyclization adapter is complementary with 3' overhang of the fragments.

In one embodiment, in 1), the DNA sequence is fragmented into random fragments by ultrasound or enzymatic cleavage.

In one embodiment, the 3' overhang of the fragments and the 5' overhang of the cyclization adapter are 1-5nt in length, e.g., 3nt or 2nt, preferably 1nt.

In one embodiment, the 3' overhang of the fragmented fragments is an A and the 5' overhang of the cyclization adapter is a T.

In one embodiment, in 2), the fragment is treated with exonuclease, polymerase and T4 polynucleotide kinase to form the 5' phosphorylated sticky end with extra A deoxynucleotide at the 3' end.

In one embodiment, in 3), the incompletely complementary double strands comprise a nick in one strand or a mis-matching region between the double strands.

In one embodiment, in 3), the two strands comprise two mis-matching regions between the double strands and include a barcode sequence for distinguishing samples between the two mis-matching regions.

In one embodiment, in 3), the cyclization adapter comprises cyclization adapter (a) or cyclization adapter (b) as follows:
the cyclization adapter (a) comprises a long strand and two short strands complementary with both ends of the long strand, wherein the long strand comprises phosphoric acid modification at the 5' end, the 5' end of the short strand complementary with the 3' end of the long strand comprises phosphoric acid modification, the cyclization adapter comprises a T-sticky end at both 3' ends and a single-stranded non-complementary region of 8-12nt (e.g., 10nt) between the complementary double strands; and preferably, the single-stranded non-complementary region comprises a barcode sequence for distinguishing samples;
the cyclization adapter (b) comprises two partially complementary strands, wherein two ends of the two strands are paired to form a double-stranded structure with phosphoric acid modification at the 5' end and a T-sticky end at the 3' end; and preferably, the double-stranded structure comprises a complementary region of 8-12nt (e.g., 10nt) as a barcode sequence for distinguishing samples.

In one embodiment, in 3), the cyclization adapter is the cyclization adapter (a), the ligated products are digested by exonuclease and the digested products are purified in one step to obtain a cyclized library.

In one embodiment, in 3), the cyclization adapter is the cyclization adapter (b) and the ligated products are denatured to obtain a cyclized library.

In one embodiment, the cyclized single-stranded library is subjected to subsequent sequencing, i.e., DNA Nanoballs (DNBs) formed by rolling circle amplication are used for reading nucleic acid sequence information.

On the other hand, the present disclosure provides a cyclization adapter for constructing cyclized library, the cyclization adapter is formed by incompletely complementary double strands and has a 5' overhang at both ends, and the 5' overhang of the cyclization adapter is complementary with the 3' overhang of the fragment to be cyclized.

In one embodiment, the 3' overhang of the fragment to be cyclized and the 5' overhang of the cyclization adapter are 1-5nt in length, e.g., 3nt or 2nt, preferably 1nt.

In one embodiment, the 3' overhang of the fragment to be cyclized is an A and the 5' overhang of the cyclization adapter is a T.

In one embodiment, the incompletely complementary double strands comprise a nick in one strand or a mis-matching region between the double strands.

In one embodiment, the two strands comprise two mis-matching regions between the double strands and include a barcode sequence for distinguishing samples between the two mis-matching regions.

In one embodiment, the cyclization adapter comprises cyclization adapter (a) or cyclization adapter (b) as follows:
the cyclization adapter (a) comprises a long strand and two short strands complementary with both ends of the long strand, wherein the long strand comprises phosphoric acid modification at the 5' end, the 5' end of short strand complementary with the 3' end of the long strand comprises phosphoric acid modification, the cyclization adapter comprises a T-sticky end at both 3' ends and a single-stranded non-complementary region of 8-12nt (e.g., 10nt) between the complementary double strands; and preferably, the single-stranded non-complementary region comprises a barcode sequence for distinguishing samples;
the cyclization adapter (b) comprises two partially complementary strands, wherein two ends of the two strands are paired to form a double-stranded structure with phosphoric acid modification at the 5' end and a T-sticky end at the 3' end and preferably, the double-stranded structure comprises a complementary region of 8-12nt (e.g., 10nt) as a barcode sequence for distinguishing samples.

The present disclosure realizes one-step cyclization of a cyclized library by using specially designed adapters. In the present disclosure, an A-sticky end is formed by end-repair and addition of an A in the fragmented DNA fragment, which is complementary with the T-sticky end of the specially designed adapter to form cyclized structure, and then the ligation at the gap is completed by ligase.

### BRIEF DESCRIPTION OF THE DRAWINGS

To illustrate the embodiments of the present disclosure or the technical solutions in the prior art more clearly, the drawings are briefly described as below. It is obvious that the drawings described below only involve some embodiments of the present disclosure.

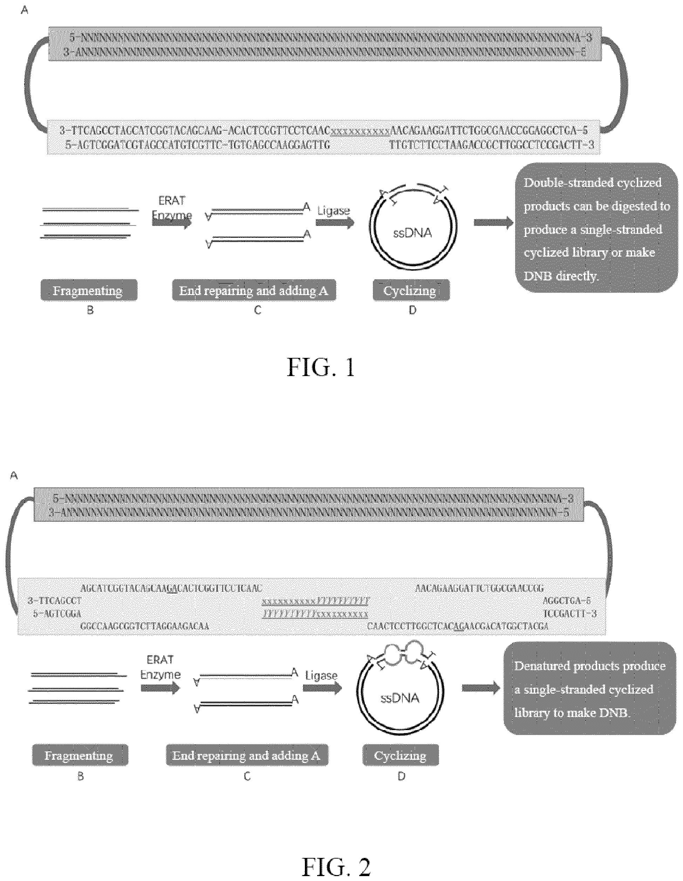

FIG. 1 shows the schematic diagram of cyclization adapter (a): A is the sequence information of the final cyclized structure in the library, the upper part is the inserted fragment, the lower part is the adapter sequence, and the underline sequence is the barcode; B, C and D are the schematic diagrams of the library construction process.
FIG. 2 shows the schematic diagram of cyclization adapter (b): A is the sequence information of the final cyclized structure in the library, the upper part is the inserted fragment, the lower part is the adapter sequence, the underline non-italic sequence is the barcode, the underline italic sequence is the sequence complementary with the specially designed barcode, and the underline sequence forms a palindromic sequence; B, C and D are the schematic diagrams of the library construction process.

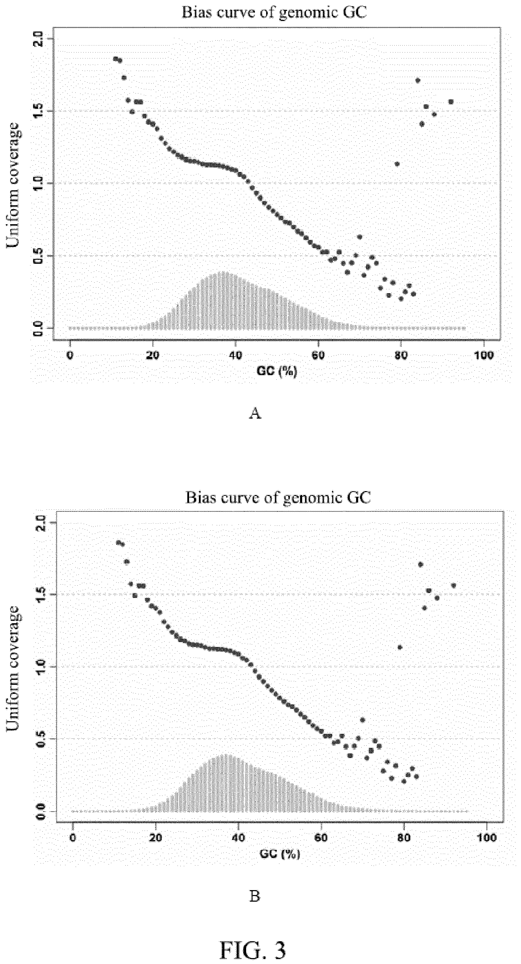

FIG. 3 shows GC coverage information in the experiment conducted with cyclization adapter (a) (A) and GC coverage information in the experiment conducted with cyclization adapter (b) (B).

### DETAILED DESCRIPTION OF THE INVENTION

The present disclosure is described clearly and completely below. It is obvious that the embodiments described are only a part of the embodiments of the present disclosure, but not all embodiments. Based on the embodiments of the present disclosure, all other embodiments obtained by those skilled in the art are within the scope of protection of the present disclosure. Unless otherwise defined, all technical and scientific terms herein have the same meanings as commonly understood by those skilled in the art to which the present disclosure belongs. The terms in the specification of the present disclosure are for the purpose of describing specific embodiments only and are not intended to limit the present disclosure.

The present disclosure provides a solution to the problems of long time-consuming of library construction, complicated operation and loss in purification in the conventional library construction process based on MGI, which includes the steps of fragmenting genomic DNA, end repair and addition of A to the end of the DNB fragments, adapter ligation, PCR amplification, separation and cyclization of a single strand, etc. Given the problems in the prior art, the inventors fundamentally improved the conventional method of adding adapters, amplification and cyclization, and designed ligation adapters with unique sequence structure, which combines the adapter ligation and product cyclization in a one-step reaction, optimizes the reaction system and purification step and significantly reduces the time of constructing a cyclized library.

As shown in FIG. 1, for the cyclization adapter (a), A shows the sequence information of the final cyclized structure in the library, the upper part shows the inserted fragment, the lower part shows the adapter sequence, and the underline sequence shows the barcode sequence; B, C and D schematically show the library construction process. The cyclization adapter (a) is formed by the complementariness of a long nucleic acid strand and two short nucleic acid strands, which allows digestion of the strand of the two short nucleic acid strands after cyclization. The length of the cyclization adapter is 84bp, and the gap between the two short nucleic acid sequences is 10bp. The long strand and the short strand at the adapter end comprise phosphoric acid modification at the 5' end. The complementary double-stranded DNA adapter comprises a sticky end of an extra T deoxynucleotide at the 3' end, and preferably, a single-stranded barcode sequence is 10bp for distinguishing samples in the middle of the adapter. The sticky end of an extra T deoxynucleotide at the 3' end of the double-stranded DNA adapter is complementarily paired with the sticky end of an extra A deoxynucleotide at the 3' end of the double-stranded DNA fragment, and then they are ligated by ligase. After ligation and cyclization, the ligated products can be digested to generate a single-stranded library or be used for DNB sequencing directly.

As shown in FIG. 2, for the cyclization adapter (b), A is the sequence information of the final cyclized structure in the library, the upper part is the inserted fragment, the lower part is the adapter sequence, the underline non-italic sequence is the barcode, the underline italic sequence is the sequence complementary with the specially designed barcode, and the underline sequence forms a palindromic sequence; B, C and D schematically show the library construction process. The cyclization adapter (b) is formed by the complementary pairing of two long nucleic acid strands with phosphoric acid modification at the 5' end. The complementary double-stranded DNA adapter comprises a sticky end of extra T deoxynucleotide at the 3' end and a non-complementary sequence in the middle, which forms a ∞ structure. The length of the cyclization adapter is 93bp. The ∞ structure formed by the non-complementary sequence in the middle enables sense strand and antisense strand of the cyclization adapter to be sequenced and reduces the formation of adapter dimers. The length of the non-complementary sequence is 34bp. The sticky end of an extra T deoxynucleotide at the 3' end of the double-stranded DNA adapter is complementarily paired with the sticky end of an extra A deoxynucleotide at the 3' end of the double-stranded DNA fragment, and then they are ligated by ligase. After ligation and cyclization, the ligated products can be denatured to generate a single-stranded library for DNB sequencing.

The innovation of the present disclosure lies in the design and construction of cyclization adapters, which are used to construct a library for double-stranded DNA by using AT ligation. The sequence design may be adapted to the MGI sequencing platform. Compared with the sequences in the conventional library construction, which have two adapters ligated to both ends of the fragment, the ligation adapter in the present disclosure can be ligated to both ends of the fragment simultaneously to realize one-step cyclization by ligation. By using the cyclization adapter in the present disclosure, the PCR and cyclization reaction in the conventional library construction can be skipped, and the purification step can be omitted, which greatly reduces the operation time of the whole library construction process. Meanwhile, the reactions of each step are ensured to proceed continuously in same tube, which avoids the operation of changing tubes and the loss in the process. Finally, a cyclized library is obtained after one-step purification.

The following examples are provided for a better understanding of the present disclosure. The experimental methods in the following examples are conventional methods, unless otherwise specified. The experimental materials in the following examples are all purchased from the regular reagent store unless otherwise specified. It should be noted that the above contents of the disclosure and the detailed description below are only for the purpose of specifically illustrating the present disclosure and are not intended to limit the present disclosure in any way. Without departing from the spirit and theme of the present disclosure, the scope of the present disclosure is determined by the appended claims.

### Examples

The sequences of the cyclization adapters in the examples are as follows:
cyclization adapter (a)
long strand a:
   5'-AGTCGGAGGCCAAGCGGTCTTAGGAAGACAAxxxxxxxxxxCAACTCCTTGGCTCAC AGAACGACATGGCTACGATCCGACTT-3', wherein xxxxxxxxxx is a barcode sequence (SEQ ID NO.1)
   short strand 1: 5'-AGTCGGATCGTAGCCATGTCGTTCTGTGAGCCAAGGAGTTG-3' (SE Q ID NO.2)
   short strand 2: 5'-TTGTCTTCCTAAGACCGCTTGGCCTCCGACTT-3' (SEQ ID NO.3) cyclization adapter (b)
long strand b: 
5'-AGTCGGAGGCCAAGCGGTCTTAGGAAGACAAxxxxxxxxxxYYYYYYYYYYCAACTC
CTTGGCTCACAGAACGACATGGCTACGATCCGACTT-3' (SEQ ID NO.4),
wherein xxxxxxxxxx is a barcode sequence and YYYYYYYYYY is the sequence complementary with the barcode.

The two strands of the cyclization adapter (b) are both long strand b, that is, the matching region and the mis-matching region at both ends of the cyclization adapter (b) are reverse to each other, and the matching region in the middle is a palindromic sequence.

### Adapter annealing:

Purchased adapters were redissolved to a concentration of 100 µM by using TE, and

For cyclization adapter (a), it was diluted according to the following formulation and allowed to stand still at room temperature for 30 minutes, to form 10 µM cyclization adapter (a):

| Long strand a (100 µM) | 10 µL |
|---|---|
| Short strand 1 (100 µM) | 10 µL |
| Short strand 2 (100 µM) | 10 µL |
| 5×STE buffer | 20 µL |
| Water | 50 µL |
| Total | 100 µL |

For cyclization adapter (b), it was diluted according to the following formulation and allowed to stand still at room temperature for 30 minutes, to form 10µM cyclization adapter (b):

| | |
|---|---|
| Long strand b (100 µM) | 20 µL |
| 5×STE buffer | 20 µL |
| Water | 60 µL |
| Total | 100 µL |

### Source:

1. Genomic DNA fragmenting: there are various methods for genomic DNA fragmenting, including physical ultrasound methods and enzyme reaction methods. There are very mature solutions for genomic DNA fragmenting in the market. A physical ultrasound fragmentation method was adopted in the example.

A 96-well PCR plate was added with a polytetrafluoroethylene thread, 1 µg of extracted genomic DNA, and TE buffer or enzyme-free water to 80 µL. The plate was sealed, and then ultrasonic fragmentation was performed on the E210 ultrasonicator.

The fragmentation condition was set as follows:

| | |
|---|---|
| Filing coefficient | 20% |
| Intensity | 5 |
| Pulse coefficient | 200 |
| Time of fragmentation | 35×4 times |

2. Fragments selecting: magnetic bead purification method or gel recycling method can be used. Magnetic bead purification method was adopted in the example. 80 µL of Ampure XP magnetic beads were added into the fragmented DNA and mixed thoroughly, and the mixture was placed for 7-15 min. The mixture was placed on a magnetic rack to collect the supernatant. 40 µL of Ampure XP magnetic beads were added into the supernatant and mixed thoroughly, and the mixture was placed for 7-15 min. The mixture was placed on a magnetic rack to remove the supernatant. The magnetic beads were washed twice with 75% ethanol, dried, added with 50 µL of TE buffer or enzyme-free water and mixed thoroughly, and the mixture was placed for 7-15 min to dissolve and recycle product.

3. End repairing and adding A: 100 ng of the product recycled in the previous step was added with TE to 40 µL and used to prepare the reaction system according to the following table. The reaction mixture was prepared as shown in the following table:

| Reagent | Volume |
|---|---|
| Nuclease-free Water | 2.1 µL |
| 10×PNK buffer | 5 µL |
| 5:1 dATP:dNTP | 0.6 µL |
| Klenow fragment | 0.1 µL |
| rTaq | 0.2 µL |
| T4 DNA polymerase | 2 µL |
| Total | 10 µL |

10 µL of end repair reaction liquid was immediately added to 40 µL of fragmented product to carry out the following reactions. The reaction conditions are shown in the following table:

| Treatment condition | Time |
|---|---|
| 37°C | 30min |
| 65°C | 15min |
| 4°C | ∞ |

4. Adapter ligating: after the reaction, 5 µL of cyclization adapter (a) or cyclization adapter (b) at a concentration of 10 µM was immediately added to the end-repaired product. The adapter ligation mixture was prepared as shown in the following table:

| Reagent | Volume |
|---|---|
| 10×PNK buffer | 3 µL |
| 100 mM ATP | 0.8 µL |
| Nuclease-free Water | 3.6 µL |
| 50% PEG8000 | 16 µL |
| T4 DNA ligase | 1.6 µL |
| Total | 25 µL |

After adding an appropriate amount of adapters into the end-repaired product, 25 µL adapter ligation mixture was added into the mixture to carry out the following reactions. The reaction conditions are shown in the following table:

| Treatment condition | Time |
|---|---|
| 23°C | 30min |
| 4°C | ∞ |

5. After the reaction, the product was purified by using 2X magnetic beads and finally dissolved in 20 µL TE solution.

6. DNB preparing and sequencing: specific steps can refer to the instruction of MGI sequencing kit. DNBs were prepared by using a double-stranded library. The preliminary results of sequencing are as follows: GC coverage information of the experiment conducted with cyclization adapter (a) is shown in A of FIG. 3, and GC coverage information of the experiment conducted with cyclization adapter (b) is shown in B of FIG. 3.

**Cyclization adapter (a)**

| Sample | Cyclization adapter a |
|---|---|
| Read number after filtering | 1076236 |
| Base number after filtering (Mb) | 161.44 |
| Proportion after filtering (%) | 52.62 |
| Mapping rate (%) | 11.1 |
| Specificity rate (%) | 98.26 |
| Duplication rate (%) | 0.83 |
| Mismatch rate (%) | 3.5 |

**Cyclization adapter (b)**

| Sample | Cyclization adapter b |
|---|---|
| Read number after filtering | 16709122 |
| Base number after filtering (Mb) | 1754.46 |
| Proportion after filtering (%) | 38.24 |
| Mapping rate (%) | 50.65 |
| Specificity rate (%) | 98.1 |
| Duplication rate (%) | 1.9 |
| Mismatch rate (%) | 1.86 |

Results analysis: given the problems in the prior art, all cyclization adapters would change the conventional method of adding adapters, amplification and cyclization, which combines the adapter ligation and product cyclization in a one-step reaction and significantly reduces the time of constructing a cyclized library. The products of library construction are cyclized, which can be directly sequenced after DNB preparation. The analysis of sequencing results shows that all adapters can be used to construct a library for sequencing successfully. Although the sequencing results are not optimal, the purpose of this experiment is to verify the feasibility of adapter design in the experiment and provide certain exemplary examples. Therefore, the results well prove that the adapter design of the present disclosure meets the requirements of the disclosure and shows certain implementability.

The specific examples are provided to describe the present disclosure, which are only to assist those skilled in the art to understand the present disclosure, but not to limit the present disclosure. Several simple deductions, variations or replacements can be made by those skilled in the art to which the present disclosure belongs according to the concept of the present disclosure.

## Claims

1. A method for constructing a cyclized library, the method comprises:
1) fragmenting a DNA sequence into fragments;
2) enabling two ends of the fragments to form 3' overhangs; and
3) cyclizing the fragments with 3' overhang to form a cyclized library by using a cyclization adapter, wherein the cyclization adapter is formed by incompletely complementary double strands and has a 5' overhang at both ends, and the 5' overhang of the cyclization adapter is complementary with the 3' overhang of the fragments.

2. The method of claim 1, the 3' overhang of the fragments is an A and the 5' overhang of the cyclization adapter is a T.

3. The method of claim 2, in 2), the fragment is treated with exonuclease, polymerase and T4 polynucleotide kinase to form the 5' phosphorylated sticky end with extra A deoxynucleotide at the 3' end.

4. The method of any one of claims 1-3, in 3), the incompletely complementary double strands comprise a nick in one strand or a mis-matching region between the double strands.

5. The method of claim 4, in 3), the two strands comprise two mis-matching regions between the double strands and include a barcode sequence for distinguishing samples between the two mis-matching regions.

6. The method of any one of claims 1-4, in 3), the cyclization adapter comprises cyclization adapter (a) or cyclization adapter (b) as follows:
the cyclization adapter (a) comprises a long strand and two short strands complementary with both ends of the long strand, wherein the long strand comprises phosphoric acid modification at the 5' end, the 5' end of the short strand complementary with the 3' end of the long strand comprises phosphoric acid modification, the complementary double-stranded adapter comprises a T-sticky end at the 3' end and a single-stranded non-complementary region of 8-12nt (e.g., 10nt); and preferably, the single-stranded non-complementary region comprises a barcode sequence for distinguishing samples;
the cyclization adapter (b) comprises two partially complementary strands, wherein two ends of the two strands are paired to form a double-stranded structure with phosphoric acid modification at the 5' end and a T-sticky end at the 3' end; and preferably, the double-stranded structure comprises a complementary region of 8-12nt (e.g., 10nt) as a barcode sequence for distinguishing samples.

7. The method of claim 6, in 3), the cyclization adapter is the cyclization adapter (a), the ligated products are digested by exonuclease, and the digested products are purified in one step to obtain a cyclized library; alternatively, the cyclization adapter is the cyclization adapter (b) and the ligated products are denatured to obtain a cyclized library.

8. A cyclization adapter for constructing cyclized library, the cyclization adapter is formed by incompletely complementary double strands and has a 5' overhang at both ends, and the 5' overhang of the cyclization adapter is complementary with the 3' overhang of the fragment to be cyclized.

9. The cyclization adapter of claim 8, the incompletely complementary double strands comprise a nick in one strand or a mis-matching region between the double strands; preferably, the two strands comprise two mis-matching regions between the double strands and include a barcode sequence for distinguishing samples between the two mis-matching regions.

10. The cyclization adapter of claim 8 or 9, the cyclization adapter comprises cyclization adapter (a) or cyclization adapter (b) as follows:
the cyclization adapter (a) comprises a long strand and two short strands complementary with both ends of the long strand, wherein the long strand comprises phosphoric acid modification at the 5' end, the 5' end of short strand complementary with the 3' end of the long strand comprises phosphoric acid modification, the complementary double-stranded adapter comprises a T-sticky end at the 3' end and a single-stranded non-complementary region of 8-12nt (e.g., 10nt); and preferably, the single-stranded non-complementary region comprises a barcode sequence for distinguishing samples;
the cyclization adapter (b) comprises two partially complementary strands, wherein two ends of the two strands are paired to form a double-stranded structure with phosphoric acid modification at the 5' end and a T-sticky end at the 3' end; and preferably, the double-stranded structure comprises a complementary region of 8-12nt (e.g., 10nt) as a barcode sequence for distinguishing samples.
